# EUROPEAN PATENT APPLICATION

(11) **EP 4 345 838 A1**
(43) Date of publication of application: **03.04.2024**
(21) Application number: 22198583.1
(22) Date of filing: 29.09.2022
(51) Int. Cl.: G16H 40/20, G16H 40/63, G02B 27/00, G02B 27/01, G06F 3/01, G06T 19/00

(54) **VISUALIZING AN INDICATION OF A LOCATION IN A MEDICAL FACILITY**

(71) Applicant: Koninklijke Philips N.V., 5656 AG Eindhoven (NL)
(72) Inventor: LAUTE, Niels, Eindhoven (NL); TUINHOUT, Jelle Jeroen, Eindhoven (NL); WIJN, Victor, 5656 AG Eindhoven (NL); BUIL, Vincentius Paulus, 5656 AG Eindhoven (NL)
(74) Representative: Philips Intellectual Property & Standards

(57) **Abstract**

The present invention relates to providing support during an activity in a medical facility. In order to provide for further improved support during activities in a medical facility, a device (10) for visualizing an indication of a location by a user in a medical facility is provided. The device comprises a data input (12), a data processor (14) and an output interface (16). The data input is configured: to provide data (18) representing a user activity of indicating a location within a medical facility; and to provide data (20) relating to a current context within the medical facility. The data processor is configured: to detect a user activity of indicating a location within a medical facility; to identify the location within the medical facility which the user is indicating; and to activate a generation of an indicator for highlighting the identified location based on the current context within the medical facility. The output interface is configured to provide the indicator (22) for highlighting the location in order to visualize the location which is indicated by the user for at least one further user.

## Description

### FIELD OF THE INVENTION

The present invention relates to providing support during an activity in a medical facility, and relates in particular to a device for visualizing an indication of a location by a user in a medical facility, to a system for providing a user's location indication for other users within a medical facility and to a method for visualizing a location indication of a user in a medical facility.

### BACKGROUND OF THE INVENTION

User interaction and communication during an activity in a medical intervention or examination is becoming increasingly essential. US 7331929 B2 describes displaying medical information based on gaze detection, wherein a direction of a user's gaze in relation to a reference position in a healthcare environment is determined. A user looking toward a display is identified based on the direction of the user's gaze in relation to the reference point in the healthcare environment to establish a user identity. Medical information displayed on the display for the user is updated based on the identity of the user. The identified user is allowed to control display of information on the display. However, it has been shown that communication may still lead to misunderstandings.

### SUMMARY OF THE INVENTION

There may thus be a need for further improved support during activities in a medical facility.

The object of the present invention is solved by the subject-matter of the independent claims; further embodiments are incorporated in the dependent claims. It should be noted that the following described aspects of the invention apply also for the device for visualizing an indication of a location by a user in a medical facility, for the system for providing a user's location indication for other users within a medical facility and for a method for visualizing a location indication of a user in a medical facility.

According to the present invention, a device for visualizing an indication of a location by a user in a medical facility is provided. The device comprises a data input, a data processor and an output interface. The data input is configured to provide data representing a user activity of indicating a location within a medical facility. The data input is also configured to provide data relating to a current context within the medical facility. The data processor is configured to detect a user activity of indicating a location within a medical facility. The data processor is further configured to identify the location within the medical facility which the user is indicating. The data processor is also configured to activate a generation of an indicator for highlighting the identified location based on the current context within the medical facility. The output interface is configured to provide the indicator for highlighting the location in order to visualize the location which is indicated by the user for at least one further user.

As an effect, user communication is facilitated. This is of advantage, for example, in situations such as where users point at certain information on screens or devices. Due to sterility constraints and hands-busy situations, users are not always able to get closer to the information they are pointing at - let alone touching the display or device. As pointing can be quite ambiguous, the present invention avoids situations of miscommunication due to user B incorrectly interpreting where user A was pointing at. This also prevents in errors, delayed workflows and is thus beneficial for inter-person communication.

According to an example, for the activation of the generation of the indicator based on the current context, the data processor is configured to provide current context data; to determine if the current context data fulfils predetermined criteria; and to activate the generation of the indicator based on the determined criteria.

According to an example, for the activation of the generation of the indicator based on the current context, the data input is configured to provide procedure data about a currently executed medical procedure. The data processor is configured to identify the situation of the user indicating the location in relation to the medical procedure; to determine whether the user activity belongs to predetermined activity scenes selected for providing the indicator to highlight the location; and to activate the generation of the indicator if the user activity belongs to the predetermined activity scenes.

According to an example, the procedure data comprises at least one of the group of: current activity parameters, context data, gestures and the indicated location.

According to an example, for the determinization, predetermined points and/or areas of interest are provided and the data processor is configured to compare the location indicated by the user to the predetermined points and/or areas to activate the generation of the indicator if the location indicated by the user matches the predetermined points and/or areas.

According to an example, for the providing of the procedure data about the current executed medical procedure, the data processor is configured to track type and phase of the current executed medical procedure.

According to an example, the data processor is configured to detect a user activity of indicating a location for at least two users; and to provide a selection of the at least two users to determine which user triggers the indicator for highlighting the location and which users are provided with a visualization of the indicator.

According to an example, the indicator for highlighting the location is provided by a head mounted device providing an extended reality view to the user wearing the head mounted device.

According to the present invention, also a system for providing a user's location indication for other users within a medical facility is provided. The system comprises a sensor arrangement, an interface arrangement, a projection arrangement and a device for visualizing an indication of a user according to any of the preceding examples. The sensor arrangement is configured to provide data representing a user activity of indicating a location within a medical facility. The interface arrangement configured to provide data relating to a current context within the medical facility. The sensor arrangement and the interface arrangement are data-connected to the device for visualizing an indication of a user. The projection arrangement is data-connected to the device for visualizing an indication of a user, and is configured to at least temporarily project the indicator to thereby highlight the location for at least one further user.

According to an example, the sensor arrangement comprises an eye tracking system; and, for the detection of the user activity of indicating a location, the data processor is configured to track the user's eye gaze and to identify a location within the medical facility at which the user is currently looking.

According to an example, the sensor arrangement comprises a gesture detecting system; and, for the detection of the user activity of indicating a location, the data processor is configured to detect a current user gesture, and to determine if the gesture comprises a pointing action comprising at least one of the group of a limb pointing action, a head pointing action or a torso action.

In an option, the data processor is configured to identify a location within the medical facility to which the user is currently pointing.

In another option, the location is identified by gaze tracking, for example exclusively, and the gesture is provided to trigger an activation of the system.

In another option, both eye gaze tracking and gesture tracking are provided to identify the location. In an example, an override function in the favor of eye gaze tracking is provided in case of a detected discrepancy between the eye gaze tracked location and the gesture tracked location.

According to an example, the projection arrangement is configured to provide the indicator for highlighting the location visually in a restricted manner to predetermined users.

In an option, the projection arrangement is configured to provide the indicator visually in a light spectrum visible to the predetermined users, but invisible to other, non-predetermined users.

According to an example, a plurality of user profiles is provided. The data processor is configured to assign the profiles to a plurality of users. The data processor is configured to identify a current context and to select a designated user for the identified context and to provide eye tracking for the designated user.

According to the present invention, also a method for visualizing a location indication of a user in a medical facility is provided. The method comprises the following steps:
- detecting a user activity of indicating a location within a medical facility;
- identifying the location within the medical facility which the user is indicating;
- activating a generation of an indicator for highlighting the identified location based on a current context within the medical facility; and
- providing the indicator for highlighting the location in order to visualize the location which is indicated by the user for at least one further user.

According to an aspect, a user pointing at a certain location within a medical space is detected. The location is then highlighted to at least one other actor.

The present invention provides improvement in terms of common usability and accessibility. It also addresses to minimize communication issues in and around cathlabs or other medical intervention rooms. A particular use scenario comprises in-lab communication between staff members and complex control rooms, and workflow challenges in hands-busy situations.

For example, the invention is of advantage in image guided therapy or when using surgical equipment during operations. In an option, the invention is applied when using augmented reality or virtual reality products

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of a device for visualizing an indication of a location by a user in a medical facility.
Fig. 2 shows an example of a system for providing a user's location indication for other users within a medical facility.
Fig. 3 shows basic steps of an example of a method for visualizing a location indication of a user in a medical facility.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

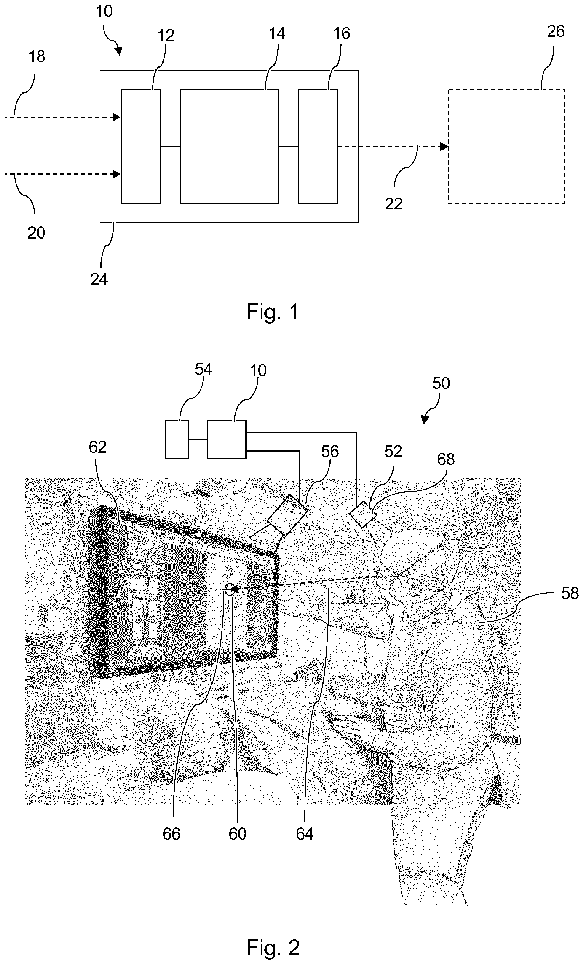

Fig. 1 schematically shows an example of a device 10 for visualizing an indication of a location by a user in a medical facility. The device 10 comprises a data input 12, a data processor 14 and an output interface 16. The data input 12 is configured to provide data representing a user activity of indicating a location within a medical facility. The data input 12 is also configured to provide data relating to a current context within the medical facility. The data processor 14 is configured to detect a user activity of indicating a location within a medical facility. The data processor 14 is also configured to identify the location within the medical facility which the user is indicating. The data processor 14 is further configured to activate a generation of an indicator for highlighting the identified location based on the current context within the medical facility. The output interface 16 is configured to provide the indicator for highlighting the location in order to visualize the location which is indicated by the user for at least one further user.

The data representing a user activity of indicating a location within a medical facility is indicated with a first arrow 18. The data relating to a current context within the medical facility is indicated with a second arrow 20. The provision of the indicator for highlighting the location in order to visualize the location which is indicated by the user for at least one further user is indicated with a third arrow 22. A frame 24 indicates the option of arranging the data input 12, the data processor 14 and the output interface 16 in a common structure, e.g. in an integrated manner. A further frame 26 indicates the representation of the indicator.

The term "user activity of indicating a location" relates to any detectable action by the user that is made in the attempt to indicate a location, i.e. point or area within the medical facility and to share this with others. The term "user activity of indicating a location" relates to a pointing action by the user to identify a desired location within the medical facility. The user activity may comprise e.g. a pointing action by the user, such as pointing with the fingertips, the hand, the elbow, the shoulder, the foot or knee or even nodding in a certain direction. The user activity may comprise e.g. that the user looks at a certain point, i.e. the pointing or identification of a certain location, or point, or spot, or area, is provided by the user's viewing action, i.e. the user's eye gaze. The indicating of a user relates to the user's attempt to identify the location within the medical facility, for example for at least one other user present in the medical facility.

The term "location" relates to any point, spot, area, region or other identifiable surface or point in space within the medical facility.

The medical facility can be an operating room, a room for medical interventions, for examinations or for treatments. For example, the medical facility is a room within a hospital. In an example, the medical facility is a cathlab (catheterization laboratory).

The term "current context" relates to the present situation in which the user is indicating the location within the medical facility. The current context may relate to e.g. a certain stage within a performed procedure. The current context may relate to e.g. a situation the user is currently in. The current context may relate to e.g. a location within the medical facility, e.g. in relation to a patient or in relation to equipment.

The "current context" data is provided in order to be considered when generating an indicator, e.g. when generating data for projecting the indicator, or at least when actually providing the indicator. The "current context" data allows to provide an indicator for the other user, or other multiple users, only when actually needed. In other words, the "current context" data enables a non-continuous, i.e. targeted presentation of the indicator. In contrast to a permanent projection of the indicator, the non-continuous manner avoids distraction or misinterpretation.

To provide the current context data and to take this data into account for generating or presenting the indicator provides a sort of intelligent indicator. This facilitates the workflow of the main user who is pointing or looking at a certain pint and who wants to show this to at least one other user. The pointing user does not need to manually switch on or switch off a pointing tool like a laser pointer. The switching-on and switching-off is provided by the device itself.

The detecting of the user activity is provided for a primary user. For example, the primary user is a predetermined user. In another example, the primary user is selected among a plurality of users.

The primary user can also be referred to as main actor.

In an example, the indicator is provided as a laser dot on the respective location intended by the user.

In an example, the indicator is provided as a spotlight.

In another example, the indicator is provided as a highlighted form of displaying on a monitor or other sort of display, e.g. when the user is identifying a certain point on a graph displayed on a monitor or the like.

In an example, for the detection of the user activity of indicating a location, the data processor is configured to track the user's eye gaze; and to identify a location within the medical facility at which the user is currently looking.

In an example, for the detection of the user activity of indicating a location, the data processor is configured to detect a current user gesture; and to determine if the gesture comprises a pointing action comprising at least one of the group of a limb pointing action, a head pointing action or a torso action; and to identify a location within the medical facility to which the user is currently pointing.

In an example, the user's eye gaze is visualized when "pointing" at a certain user interface, data point, device or other point or area of interest. Using eye gaze tracking and/or hand posture tracking, the provided system enables a user B to see the eye gaze of user A when pointing at a point of interest that is relevant for the current workflow step or conversation. In an example, for a situation where a user wants to point out a point of interest to others, while not being able to manually point at it due to a both hands busy situation, e.g. when manipulating a guidewire, catheter or a device, it is provided to activate "pointing with the eyes" through another means in such situations.

The term "data input" relates to providing or supplying data for data processing steps. The data input can also be referred to as image data input. The data input can also be referred to as data supply, as image data supply, as image input, as input unit or simply as input. In an example, the image data input is data-connectable to an imaging source arrangement.

The term "data processor" relates to a processor or part of a processor arrangement that is provided to conduct the computing steps using the data supplied by the data input. The data processor can also be referred to as data processing arrangement, as processor unit or as processor. In an example, the data processor is data-connected to the data input and the output interface.

The term "output interface" relates to an interface for providing the processed or computed data for further purposes. The output interface can also be referred to as output or output unit. In an example, the output interface is data-connectable to a display arrangement or display device. In another example, the output is data-connected to a display. As an example, the signals by the controller can be provided by the output interface.

In an example, not further shown in detail, for the activation of the generation of the indicator based on the current context, the data processor 14 is configured:
- to provide current context data;
- to determine if the current context data fulfils predetermined criteria; and
- to activate the generation of the indicator based on the determined criteria.

In an example, not further shown in detail, for the activation of the generation of the indicator based on the current context, the data input 12 is configured to provide procedure data about a currently executed medical procedure. The data processor 14 is configured to identify the situation of the user indicating the location in relation to the medical procedure. The data processor 14 is also configured to determine whether the user activity belongs to predetermined activity scenes selected for providing the indicator to highlight the location. The data processor 14 is further configured to activate the generation of the indicator if the user activity belongs to the predetermined activity scenes.

In an option, the data processor 14 is configured to understand the current context and to control the triggering of the generation of the indicator accordingly, e.g. non-continuously.

In an example, not further shown in detail, the procedure data comprises at least one of the group of: current activity parameters, context data, gestures and the indicated location.

In an example, not further shown in detail, for the determinization, predetermined points and/or areas of interest are provided and the data processor 14 is configured to compare the location indicated by the user to the predetermined points and/or areas to activate the generation of the indicator if the location indicated by the user matches the predetermined points and/or areas.

In an example, the predetermined points and/or areas of interest are provided per medical procedure step.

In an example, the points and areas of interest are related to a certain point of time of interest of the procedure.

In an example, the points and areas of interest are related to a certain step of interest of the procedure.

The medical procedure can also be referred to as medical workflow. The medical procedure step can also be referred to as medical workflow step.

The term "procedure data" relates to all information that allows to retrieve, i.e. to determine the currently performed procedure. The procedure data may comprise information about a current personnel scenery, medical equipment that allows identification about the performed tasks, subject or staff data, and any other features.

As a sort of start, an understanding of a currently performed procedure and a current scenario or situation in an operating room is provided. This understanding is taken to determine possible further scenarios or situations in view of the conducted medical procedure, like an operation. In other words, the previous procedure is understood and, optionally, the further procedure is predicted.

The understanding may be provided by respective data. In an option, the understanding is generated by detecting the current situation, e.g. by optical cameras. Further, also other data sources like the conversation among the staff members or stored information about the subject and the planned procedure are used for achieving the understanding of the current situation in the context of the respective procedure.

In an example, the medical procedure takes place in an operational room.

In an example, in addition to position determination of staff and equipment, movement patterns are tracked to determine a current executed medical procedure, to provide the procedure data.

In an example, not further shown in detail, for the providing of the procedure data about the current executed medical procedure, the data processor 14 is configured to track type and phase of the current executed medical procedure.

The term "type" relates to the kind or category of the medical procedure.

The term "phase" relates to a certain identifiable part, portion or sequence within the medical procedure.

In an option, for the tracking of the type and phase of the current executed medical procedure, the data processor is configured to provide at least one of the group of:
- procedure analysis based on image data of a current scenery; and
- procedure analysis based on acoustic data comprising at least parts of a conversation by the staff of a current scenery; and patient related data.

In an example, the patient related data comprises the type of operation currently performed. In another example, the patient related data comprises current, i.e. updated data, that allows retrieving the phase of the operation currently performed. As an example for updated data, medical data of the patient is monitored, or equipment activity is monitored such that a documentation of certain operational steps allows retrieving the phase of the operation currently performed.

In an example, this also includes input on the procedure data in the form of knowledge. As an example, an input is provided about what procedure is done at the moment, e.g. from electronic medical record (EMR), and what such procedure constitutes of, e.g. which steps. In an option, this can also be stored in e.g. a database. In an option, the image and acoustic data is then used to recognize transitions in steps of the procedure.

In an example, procedure data is provided before a procedure is started and the available equipment is tracked. Based on equipment movement and staff instructions, an understanding of the operation procedure is provided. Further, as the procedure is started and is going on, the procedure data is provided at least in regular intervals, or even continuously.

In an example, not further shown in detail, the data processor 14 is configured to detect a user activity of indicating a location for at least two users. The data processor 14 is configured to provide a selection of the at least two users to determine which user triggers the indicator for highlighting the location and which users are provided with a visualization of the indicator.

In an example, different indicator visualizations are provided to achieve a visual differentiation of respective users' indication of the location. In an example, several users with different roles are provided. A first database is provided with users, roles and permissions stored therein. This data is used for user identification per user. A second database is provided with procedure workflow data stored therein. Further, eye gaze tracking sensors (or other pointer sensors) are provided per user. Still further, data about point of interest, such as application window, control panel or surgery area is provided. The user identification, the tracking data from the sensors and the point of interest data is used for a gaze point sharing service. This service is also provided with data from the first database, and with data relating to workflow steps, such as imaging data, process phase and interaction behavior, and data from the second database. The gaze point sharing service then provides interactor gaze point visualization per user.

In another example, gaze point sharing of main interactor is provided for a workflow task for more than one other user. In a first decision step, it is divided into gaze indication, or pointing indication, required for person looking at the same information and gaze indication, or pointing indication, not required for person looking at the same information. For the required gaze indication, a gaze position is visualized to the other person. For the non-required gaze indication, the gaze position is not visualized to the other person.

In an example, not further shown in detail, the indicator for highlighting the location is provided by a head mounted device providing an extended reality view to the user wearing the head mounted device.

Extended reality (XR) comprises e.g. mixed reality, augmented reality and virtual reality.

In an example, the head mounted device provides a virtual reality (VR) presentation to the user, in which a virtual view is provided plus the indicator which is at least temporarily shown depending on the main user's activity and activation of indicating the location.

In another example, the head mounted device provides a mixed reality (MR) presentation to the user, in which artificial projections are provided overlaid with a user's view on the real environment plus the indicator which is at least temporarily shown depending on the main user's activity and activation of indicating the location.

In an option, a combination of VR, MR, and AR presentation forms is provided, for example for different users or different user groups.

In a further example, mixed reality is provided, such as a display arrangement showing a view of the real environment, but enriched with additional information overlaid to the real image. Mixed reality as an emerging technology can play a substantial role in the cathlab to simplify procedures and improve the overall user experience. Mixed reality also facilitates providing new ways of communicating, e.g. remote collaboration.

In an example, MR headsets are provided with a wide array of sensors that can capture the human senses. This also enables new interaction paradigms, such as eye gaze tracking or voice commands. Additionally, the output of these senses is captured and visualized for other users in the same physical room or remote.

In a further example, provided alternatively or in addition to the head mounted device providing the indicator to a (secondary) user, a head mounted device is provided for the eye gaze tracking of the (main) user.

As an example, a foot controller or other means are provided to turn on or off the users' eye gaze when pointing.

In order to save time, e.g. when the user's eye gaze could unintentionally switch between points of interest in a rapid pace, without the user actively doing this, the context based activation is provided. This avoids distracting features for other users in the room.

As another example, for cases where the user's eye gaze should not be visualized for all persons in the room, e.g. based on certain criteria or role definitions, an individualized visualization is provided. This can also be finetuned based on the workflow step, so in some cases the pointer might (not) activate if it does not provide any value to the users in the current workflow step.

Showing the user's gaze in a targeted way, i.e. non-continuously, is useful in situations where the user's eye gaze should not be visible to others, as eye gaze visualization can be e.g. privacy invasive. As an example, other users should not be able to see the user's eye gaze, e.g. patient. Further, eye gaze visualization can be distracting, e.g. unexpected eye gaze movement might be distracting to other users. Furthermore, eye gaze visualization can be unintentional, e.g., users may be staring at the ceiling when thinking.

In an option, the system automatically recognizes when it is relevant to automatically activate gaze visualization during a cathlab intervention.

In an additional option, the system is configured to intelligently turn off the laser pointer (or other indicator projection device) based on the workflow step or context in the lab. As an example, the pointer is disabled automatically whenever it is no longer relevant for the receiving person or does not add relevance to the workflow step.

Fig. 2 shows an example of a system 50 for providing a user's location indication for other users within a medical facility. The system 50 comprises a sensor arrangement 52, an interface arrangement 54, a projection arrangement 56 and an example of the device 10 for visualizing an indication of a user according to any of the preceding examples. The sensor arrangement 52 is configured to provide data representing a user activity of indicating a location within a medical facility. The interface arrangement 54 is configured to provide data relating to a current context within the medical facility. The sensor arrangement 52 and the interface arrangement 54 are data-connected to the device 10 for visualizing an indication of a user. Further, the projection arrangement 56 is data-connected to the device 10 for visualizing an indication of a user. The projection arrangement 56 is configured to at least temporarily project the indicator to thereby highlight the location for at least one further user.

In an option, for example, the projection arrangement 56 is provided comprising an actively projecting device, i.e. a separate projection arrangement. In an example, a light source is provided for projecting a respective indicator on a designated surface at the identified location. In another example, a light source is provided for projecting a respective indicator on a designated surface such that the user, for which the indicator is intended, can see the indicator matching with the identified location, such as in an extended reality projection, e.g. augmented reality.

In another option, for example, the projection arrangement 56 is provided as controlling the projection of data, for example on a monitor or display surface such that the shown content also comprises the indicator. In other words, the projection arrangement 56 is provided as a passive device, but not a separate projection arrangement, while using another existing actively projecting device like a monitor.

As an example, a first user 58 is indicated who is looking at a certain point 60 on a monitor 62 within an operating room. A broken line 64 indicates the eye gaze.

The projection arrangement 56 may provide a projection 66 of an indicator to highlight the point 60.

In an example, not further shown in detail, the sensor arrangement 52 comprises an eye tracking system 68. For the detection of the user activity of indicating a location, the data processor 14 is configured to track the user's eye gaze, and to identify a location within the medical facility at which the user is currently looking.

In an example, provided alternatively or in addition, but not further shown in detail, the sensor arrangement 52 comprises a gesture detecting system. For the detection of the user activity of indicating a location, the data processor is configured: to detect a current user gesture; to determine if the gesture comprises a pointing action comprising at least one of the group of a limb pointing action, a head pointing action or a torso action; and to identify a location within the medical facility to which the user is currently pointing.

The gesture can also be referred to as user handling or user action.

In an option, only the gesture detecting is provided.

In another option, only the eye gaze detecting is provided.

In a further option, the eye gaze detecting is provided plus the gesture detecting.

In an example, not further shown in detail, the projection arrangement 56 is configured to provide the indicator for highlighting the location visually in a restricted manner to predetermined users.

In an option, the projection arrangement 56 is configured to provide the indicator visually in a light spectrum visible to the predetermined users, but invisible to other, non-predetermined users.

In an example, not further shown in detail, a plurality of user profiles is provided. The data processor 14 is configured to assign the profiles to a plurality of users; and the data processor 14 is configured to identify a current context and to select a designated user for the identified context and to provide eye tracking for the designated user.

As an example, the indicator is provided with a predetermined laser wave spectrum, whereas the selected predetermined users or the non-selected predetermined users are provided with viewing filters.

In an example, ceiling-mounted or headset-mounted laser pointer systems are provided to generate the indicators.

As another example, users are equipped with MR, AR or VR viewing devices and the indicator is provided as projection within the field of view of the MR, AR or VR viewing devices.

In an example, the indicator for highlighting the location is visually provided only to pre-selected users. In an example, the indicator for highlighting the location is visually provided only to one user. The user can also be referred to as secondary user or passive user.

In an option, a system is provided with sensors that are able to understand the workflow of multiple users in a cathlab. As an example, eye gaze trackers are provided that are capable of detecting the user's gaze. Based on the workflow step, and optionally other criteria, the system can decide to visualize the user's gaze to other team members looking at the same point of interest. The system can also decide to hide the user's gaze if certain criteria, e.g. privacy or relevancy, are not met.

In an option, user identification and a role and/or permission database is provided.

As an example, hardware and software sensors are provided that are capable of detecting who is in the room and what their profile is. This profile contains information on role, permissions and other relevant, personal data. As an option, certain roles get personalized indication highlights in specific situations when it is relevant for a specific role to look at a particular area.

In an option, eye gaze tracking sensors are provided.

As an example, the eye gaze of each user is tracked by either using a head mounted device, e.g. MR device, or sensors integrated in user-facing equipment, e.g. the boom monitor. It should be able to track the user's eye gaze position in space.

In an option, a workflow step understanding is provided.

As an example, by leveraging multiple data sources in the lab, e.g. data that is captured by the lab itself, EMRs, and using optical image recognition, the system understands in what step of the workflow users are while executing the procedure.

In an option, a point of interest capturing is provided.

As an example, based on the workflow understanding, points of interests are defined per workflow step. For example, guiding the catheter through the guidewire could mark the X-ray application on the boom monitor as a point of interest. Other, non-essential but still relevant data such as hemo values, would be marked with a lower point of interest score. Other elements in the room (e.g. a personal phone screen) is marked as "private" and should not be marked as a point of interest.

In an option, a gaze point visualization service is provided.

As an example, by combining the before mentioned elements, the system is capable to detect that: Users are discussing a certain visible application, item or other object in the room. The object would be marked as relevant for the current workflow step. The system would detect two or more users are looking at the same object. The gaze of the main actor, e.g. the head surgeon, is visualized on the object (e.g. on the 2D display or mixed reality) and visible to all other users that meet the role / permissions criteria.

If permissions are not met, or if the gaze is no longer on the active focus (a slight delay can be considered, to avoid rapid changes), the active gaze visualizer will be disabled and no longer be visible.

As an example, the following use case scenario is provided: At the start of the procedure, the staff members in the cathlab are identified and their roles / permissions in the lab are known to the system. This can be done by using facial recognition to detect who entered the room and be tracked at all times; or through a personal device the user would be wearing (e.g. an AR headset or other means of wearable device). During the procedure, the users eye gaze is tracked through integrating an eye gaze sensor in obvious "points of interests" (e.g. the boom monitor screen), and/or integrated into a personal wearable device (e.g. AR headset or lead glasses). During the procedure, a system is capable of detecting key events through the workflow. This can be done by using information captured by data sources connected to the cathlab, such as a PACS system, EMR, or by software that is actively processing information captures during the procedure. Or algorithms are provided that are capable of combining these data sources and detecting procedure steps so the system is capable of detecting staff member intentions related to the procedure. Audio could be captured and using natural language processing (NLP) to understand what was mentioned in the cathlab. These key events can be used to determine if there is a communication, efficiency or clinical benefit to visualize the users eye gaze on the display for others to see. The visualization service can translate the X,Y positions of the user and visualize on top of software applications to provide additional context to other staff members.

In an example, the context of use, and personal preferences of the main actor, are used to determine if pointer should be disabled whenever that is deemed appropriate by the system. For example, the pointer is disabled automatically when looking at an object, person, or region that is not relevant to the current workflow step.

The indicator for highlighting the location in order to visualize the location which is indicated by the user for at least one further user is provided in a selected manner depending on a current context.

In an example, the user's gaze is visualized in mixed reality. This has multiple benefits: The gaze pointer is visualized throughout the entire room, going beyond the boundaries of a 2D display. The gaze pointer is rendered on each individual device that is in the room. Therefore, each gaze pointer is unique to each user wearing the headset. The position and representation can be tweaked and optimized for each individual user. Profile information can be used to determine if a user has the right permissions to see the gaze pointer - or if there are clear benefits to do so, e.g. a supporting staff member is not part of the clinical decision process, and has no clear benefit to see the gaze pointer of the surgeon whereas a fellow physician does. Certain points of interests throughout the room can be marked as "private" or not relevant to visualize to others.

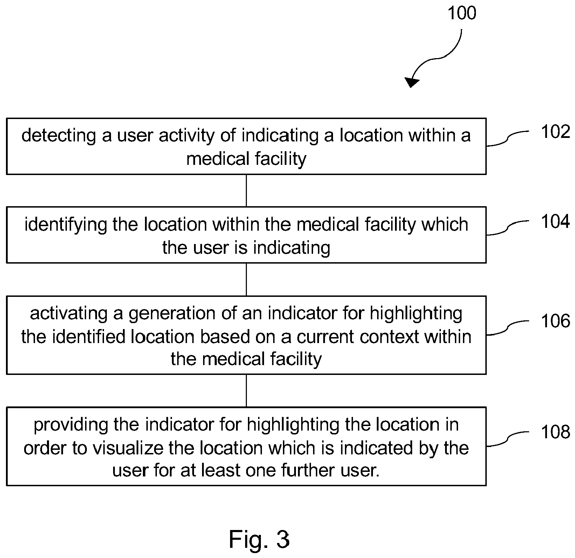

Fig. 3 shows basic steps of an example 100 of a method for visualizing a location indication of a user in a medical facility. The method 100 comprises the following steps: In a first step 102, a user activity of indicating a location within a medical facility is detected. In a second step 104, the location within the medical facility which the user is indicating is identified. In a third step 106, a generation of an indicator for highlighting the identified location is activated based on a current context within the medical facility. In a fourth step 108, the indicator for highlighting the location is provided in order to visualize the location which is indicated by the user for at least one further user.

In an example of the method, the detection of the user activity of indicating a location comprises the steps of:
- tracking the user's eye gaze; and
- identifying a location within the medical facility at which the user is currently looking. In an example of the method, the detection comprises the steps of:
- detecting a current user gesture;
- determining if the gesture comprises a pointing action comprising at least one of the group of a limb pointing action, a head pointing action or a torso action; and
- identifying a location within the medical facility to which the user is currently pointing.

In an example of the method, for the activating of the generation of the indicator based on the current context, it is provided the step of:
- providing current context data;
- determining if the current context data fulfils predetermined criteria; and
- activating the generation of the indicator based on the determined criteria.

In an example of the method, for the activating of the generation of the indicator based on the current context, it is provided the steps of:
- providing procedure data about a current executed medical procedure;
- identifying the situation of the user indicating the location in relation to the medical procedure;
- determining whether the user activity belongs to predetermined activity scenes selected for providing the indicator to highlight the location; and
- activating the generation of the indicator if the user activity belongs to the predetermined activity scenes.

In an example of the method, the procedure data comprises at least one of the group of: current activity parameters, context data, gestures and the indicated location.

In an example of the method, for the determining, predetermined points and/or areas of interest are provided and the location indicated by the user is compared to the predetermined points and/or areas to activate the generation of the indicator if the location indicated by the user matches the predetermined points and/or areas.

In an example of the method, for the providing of the procedure data about the current executed medical procedure, the data processor tracks type and phase of the current executed medical procedure.

In an example of the method, for the tracking of the type and phase of the current executed medical procedure, it is provided at least one of the group of: procedure analysis based on image data of a current scenery, procedure analysis based on acoustic data comprising at least parts of a conversation by the staff of a current scenery, and patient related data.

In an example of the method, a user activity of indicating a location is detected for at least two users. A selection of the at least two users is provided to determine which user triggers the indicator for highlighting the location and which users are provided with a visualization of the indicator.

In an example of the method, the indicator for highlighting the location is visually provided in a restricted manner to predetermined users. In an option, the indicator is visually provided in a light spectrum visible to the predetermined users, but invisible to other, non-predetermined users.

In an example of the method, the indicator for highlighting the location is provided by a head mounted device providing an extended reality view to the user wearing the head mounted device.

According to an example, a computer program is provided that comprises instructions which, when the program is executed by a computer, cause the computer to carry out the method of one of the previous examples.

In an example, a computer program or program element for controlling an apparatus according to one of the examples above is provided, which program or program element, when being executed by a processing unit, is adapted to perform the method steps of one of the method examples above.

In another example, a computer readable medium is provided having stored the computer program of the preceding example.

The term "subject" may also be referred to as individual. The "subject" may further also be referred to as patient, although it is noted that this term does not indicate whether any illness or disease is actually present with the subject.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit or be distributed over more than one computer units, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

This exemplary embodiment of the invention covers both, a computer program that right from the beginning uses the invention and a computer program that by means of an update turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A device (10) for visualizing an indication of a location by a user in a medical facility, the device comprising:
- a data input (12);
- a data processor (14); and
- an output interface (16);
wherein the data input is configured:
- to provide data (18) representing a user activity of indicating a location within a medical facility; and
- to provide data (20) relating to a current context within the medical facility;
wherein the data processor is configured:
- to detect a user activity of indicating a location within a medical facility;
- to identify the location within the medical facility which the user is indicating; and
- to activate a generation of an indicator for highlighting the identified location based on the current context within the medical facility; and
wherein the output interface is configured:
- to provide the indicator (22) for highlighting the location in order to visualize the location which is indicated by the user for at least one further user.

2. Device according to claim 1, wherein, for the activation of the generation of the indicator based on the current context, the data processor is configured:
- to provide current context data;
- to determine if the current context data fulfils predetermined criteria; and
- to activate the generation of the indicator based on the determined criteria.

3. Device according to claim 1 or 2, wherein, for the activation of the generation of the indicator based on the current context, the data input is configured to provide procedure data about a currently executed medical procedure;
and the data processor is configured:
- to identify the situation of the user indicating the location in relation to the medical procedure;
- to determine whether the user activity belongs to predetermined activity scenes selected for providing the indicator to highlight the location; and
- to activate the generation of the indicator if the user activity belongs to the predetermined activity scenes.

4. Device according to claim 1, 2 or 3, wherein the procedure data comprises at least one of the group of: current activity parameters, context data, gestures and the indicated location.

5. Device according to one of the preceding claims, wherein, for the determinization, predetermined points and/or areas of interest are provided and the data processor is configured to compare the location indicated by the user to the predetermined points and/or areas to activate the generation of the indicator if the location indicated by the user matches the predetermined points and/or areas.

6. Device according to one of the preceding claims, wherein, for the providing of the procedure data about the current executed medical procedure, the data processor is configured to track type and phase of the current executed medical procedure.

7. Device according to one of the preceding claims, wherein the data processor is configured:
- to detect a user activity of indicating a location for at least two users; and
- to provide a selection of the at least two users to determine which user triggers the indicator for highlighting the location and which users are provided with a visualization of the indicator.

8. Device according to one of the preceding claims, wherein the indicator for highlighting the location is provided by a head mounted device providing an extended reality view to the user wearing the head mounted device.

9. A system (50) for providing a user's location indication for other users within a medical facility, the system comprising:
- a sensor arrangement (52);
- an interface arrangement (54);
- a projection arrangement (56); and
- a device (10) for visualizing an indication of a user according to any of the preceding claims;
wherein the sensor arrangement is configured to provide data representing a user activity of indicating a location within a medical facility;
wherein the interface arrangement configured to provide data relating to a current context within the medical facility;
wherein the sensor arrangement and the interface arrangement are data-connected to the device for visualizing an indication of a user; and
wherein the projection arrangement is data-connected to the device for visualizing an indication of a user, and is configured to at least temporarily project the indicator to thereby highlight the location for at least one further user.

10. System according to claim 9, wherein the sensor arrangement comprises an eye tracking system (68); and
wherein, for the detection of the user activity of indicating a location, the data processor is configured:
- to track the user's eye gaze; and
- to identify a location within the medical facility at which the user is currently looking.

11. System according to claim 9 or 10, wherein the sensor arrangement comprises a gesture detecting system; and
wherein, for the detection of the user activity of indicating a location, the data processor is configured:
- to detect a current user gesture;
- to determine if the gesture comprises a pointing action comprising at least one of the group of a limb pointing action, a head pointing action or a torso action; and
- to identify a location within the medical facility to which the user is currently pointing.

12. System according to claim 9, 10 or 11, wherein the projection arrangement is configured to provide the indicator for highlighting the location visually in a restricted manner to predetermined users; and
wherein the projection arrangement is configured to provide the indicator visually in a light spectrum visible to the predetermined users, but invisible to other, non-predetermined users.

13. System according to one of the claims 9 to 12, wherein a plurality of user profiles is provided;
wherein the data processor is configured to assign the profiles to a plurality of users; and
wherein the data processor is configured to identify a current context and to select a designated user for the identified context and to provide eye tracking for the designated user.

14. A method (100) for visualizing a location indication of a user in a medical facility, the method comprising the following steps:
- detecting (102) a user activity of indicating a location within a medical facility;
- identifying (104) the location within the medical facility which the user is indicating;
- activating (106) a generation of an indicator for highlighting the identified location based on a current context within the medical facility; and
- providing (108) the indicator for highlighting the location in order to visualize the location which is indicated by the user for at least one further user.

15. Computer program comprising instructions which, when the program is executed by a computer, cause the computer to carry out the method of claim 14.
